(19) Europäisches Patentamt
European Patent Office
Office européen des brevets

(11) Veröffentlichungsnummer: **0 226 715**
**B1**

(12) **EUROPÄISCHE PATENTSCHRIFT**

(45) Veröffentlichungstag der Patentschrift: **28.11.90**

(21) Anmeldenummer: **86112281.0**

(22) Anmeldetag: **05.09.86**

(51) Int. Cl.[5]: **C 02 F 3/30,** C 02 F 1/20, C 02 F 3/34

# (54) Verfahren zur biologischen Denitrifizierung von Wasser und Vorrichtung zur Durchführung dieses Verfahrens.

(30) Priorität: **28.09.85 DE 3534716**
**19.12.85 DE 3545020**

(43) Veröffentlichungstag der Anmeldung:
**01.07.87 Patentblatt 87/27**

(45) Bekanntmachung des Hinweises auf die Patenterteilung:
**28.11.90 Patentblatt 90/48**

(84) Benannte Vertragsstaaten:
**AT BE CH DE FR GB IT LI LU NL SE**

(56) Entgegenhaltungen:
**DE-A-2 316 483**
**DE-A-2 616 212**
**DE-B-1 201 262**
**FR-A-2 189 328**
**FR-A-2 342 251**
**FR-A-2 518 078**
**US-A-4 017 276**

(73) Patentinhaber: **Eppler, Alwin**
**Gartenstrasse 9**
**D-7295 Dornstetten (DE)**
(73) Patentinhaber: **Eppler, Dieter**
**Gartenstrasse 9**
**D-7295 Dornstetten (DE)**

(72) Erfinder: **Eppler, Alwin**
**Gartenstrasse 9**
**D-7295 Dornstetten (DE)**
Erfinder: **Eppler, Dieter**
**Gartenstrasse 9**
**D-7295 Dornstetten (DE)**

(74) Vertreter: **Patentanwälte Kohler - Schwindling - Späth**
**Hohentwielstrasse 41**
**D-7000 Stuttgart 1 (DE)**

**EP 0 226 715 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) References cited:

Chemical Abstracts, Band 88, Nr. 6, 06.Febr. 1988, Seite 221, Zusammenfassung Nr. 41265a, Columbus, Ohio, US; K. SHIMAHARA et al.: "Utilization of microorganisms for cleanup of nitrogen oxides. Part 1. Isolation and identification of bacteria denitrifying nitrate and nitrite" & SEIKEI DAIGAKU KOGAKUBU KOGAKU HOKOKU, 1977, 24, 1687-94

Chemical Abstracts, Band 98, Nr. 14, 04. April 1983, Seite 348, Zusammenfassung Nr. 113141p Columbus, Ohio, US; & JP A 5742395

**Beschreibung**

Die Erfindung betrifft ein Verfahren zur biologischen Denitrifizierung von Wasser zur Aufbereitung von Trinkwasser unter Verwendung eines Schwebe- oder Wirbelbettreaktors mit einer Denitrifikationsstufe, die ein Trägermaterial mit darauf angesidelten heterotrophen Denitrifikationsbakterien enthält, bei welchem Verfahren das aufzubereitende Wasser mit Nährstoffen für die Denitrifikationsbakterien versetzt und dann durch die Denitrifikationsstufe hindurchgeleitet wird.

Im Hindblick auf die zunehmende Verunreinigung von Grund- und Oberflächenwässern mit Nitraten kommt der Denitrifizierung von Trinkwasser eine zunehmende Bedeutung zu. Neben Ionenaustausch und Umkehrosmose kommt hierfür auch die biologische Denitrifizierung in Frage.

Aus CHEM. ABSTRACTS, Band 98, Nr., 14, Seite 348, Nr. 113 141p ist ein Verfahren zur Nitrifikation/Denitrifikation von Abwasser bekannt, wobei vor der Denitrifikation $O_2$ eliminiert wird. Durch das $O_2$-Eliminationsbecken wird $N_2$ durchgeleitet.

Die FR—A—2 342 251 offenbart ein Verfahren zur biologischen Abwasser-Reinigung, wobei zwischen der biologischen Berhandlung und der Nachklärung eine Vakuumentgasung durchgeführt wird.

Die Aufgabe dieses Verfahrens ist es, die Nachklärung durch die Beseitigung der störenden Gase, die während der vorhergehenden Belüftung in das Wasser eingeleitet wurden, zu verbessern.

Aus der DE—A—2 616 212 ist ebenfalls ein Verfahren zur biologischen Abwasserreinigung bekannt. Eine $O_2$-Eliminierung wird in einem offenen Becken vor der Denitrifizierung vorgenommen.

Die genannten Verfahren sind zur Aufbereitung von Trinkwasser ungeeignet.

Die Erfindung geht von einem Verfahren zur biologischen Denitrifizierung von Wasser aus, wie es von Haberer in der DVGW-Schriftenreihe "Wasser" Nr. 31, Eschborn 1982, Seiten 147 bis 162, insbesondere Seite 152 ff., beschrieben ist.

Bei der Trinkwasseraufbereitung spielen die Verfahrenskosten eine erhebliche Rolle, da stets sehr große Wassermengen zu denitrifizieren sind und die Aufbereitung von Trinkwasser keine große Kosten verursachen soll. Bei den Denitrifizierungsverfahren fallen u.a. die Kosten für die zur Ernährung der Bakterien einzusetzenden Nährstoffe ins Gewicht. Als Nährstoffe finden beispielsweise Alkohole, Melasse u. dgl. Verwendung. Darüber hinaus spielt auch die Zeit eine Rolle, die benötigt wird, bis eine Denitrifikationsstufe nach Start des Verfahrens ihre volle Wirksamkeit erreicht. Diese Zeit ist insbesondere dann von Bedeutung, wenn die Denitrifikationsstufe in kürzeren Zeitabständen stillgelegt werden muß, um durch das ständige Wachstum der Biomase verstopfte Kanäle für den Wasserdurchtritt wieder freizumachen.

Demgemäß liegt der Erfindung die Aufgabe zugrunde, ein Verfahren nach dem Oberbegriff des Anspruches 1 so zu verbessern, daß sowohl ein verminderter Einsatz an Nährstoffen für die Bakterien ausreicht als auch die Verfahrensbedingungen im Sinne einer Absenkung der Betreibskosten verbessert werden.

Diese Aufgabe wird nach der Erfindung dadurch gelöst, daß das Wassers vor dessen Hindurchleiten durch die Denitrifikationsstufe einer Vakuumentgasung unterworfen wird.

Die Denitrifikationsbakterien verbrauchen zunächst den im Wasser gelösten Sauerstoff, bevor sie das im Wasser enthaltene Nitrat aufspalten, um den an dem Stickstoff gebundenen Sauerstoff zu verbrauchen. Es mußten daher stets zusätzlich so viel Nährstoffe zugeführt werde, wie erforderlich waren, um den im Wasser gelösten Sauerstoff zu verbrauchen. Diese Menge an Nährstoffen wird bei dem erfindungsgemäßen Verfahren eingespart, da durch die vorgeschaltete Vakuumentgasung der Sauerstoffgehalt des Rohwassers reduziert wird, und es wird eine entsprechende Senkung der Betriebskosten erzielt.

Darüber hinaus ergeben sich durch die erfindungsgemäße Vakuumentgasung des Rohwassers noch erhebliche weitere Vorteile. Durch den Verbrauch des im Wasser gelösten Sauerstoffes findet eine Vermehrung der Bakterien statt, die zu einem übermäßrigen Anwachsen der Biomasse führt, ohne daß dadurch die Denitrifizierung verbessert wird, und zwar gerade auch von Bakterien, die zu einer Nitratspaltung nicht fähig sind und daher die Wirksamkeit der Biomasse beeinträchtigen. Daher kommt es beim Anfahren eines Reaktors nur sehr langsam zur Ausbildung einer ausreichenden Menge an Denitrifikationsbakterien und infolgedessen nur sehr langsam zur vollen Aktivität des Reaktors. Weiterhin verursacht die für die Denitrifizierung unwirksame Biomasse ein vorschnelles Verstopfen der in der Denitrifikationsstufe vorhandenen engen Zwischenräume, so daß eine häufige Betriebsunterbrechung zum Zweck der Reinigung mit anschließender längerer Anfahrzeit erforderlich ist. Die Häufigkeit und die Dauer der Betriebsunterbrechungen wird durch die Reduzierung des Sauerstoffgehaltes des Rohwassers aufgrund der erfindungsgemäßen Vakuumentgasung vermindert, was ebenfalls zur Wirtschaftlichkeit des Verfahrens beiträgt.

Ein weiterer Vorteil des erfindungsgemäßen Verfahrens besteht darin, daß die Bildung von unerwünschtem Nitrit vermindert wird, das sich sonst leicht bei Vorliegen von im Wasser gelöstem Sauerstoff bildet. Endlich erzeugen die Bakterien aus dem zugeführten Nährstoff und dem im Wasser vorliegenden Sauerstoff Kohlensäure, die ebenfalls die Wirksamkeit der Denitrifizierung beeinträchtigt.

Ein weiterer Vorteil besteht darin, daß durch zuführen von Wasser mit geringem Sauerstoffgehalt in der Denitrifikationsstufe keine Zone entsteht, in welcher zunächst der im Wasser vorhandene Sauerstoff verbraucht wird, so daß die Größe der Denitrifikationsstufe um das Ausmaß dieser sonst benötigten Zone vermindert werden kann. Auch hierdurch wird die Wirtschaftlichkeit des Verfahrens erhöht.

Die erfindungsgemäße Vakuumentgasung ist ohne großen apparativen Aufwand und auch ohne übermäßigen Energieeinsatz möglich. Damit läßt sich eine Reduktion des Sauerstoffgehaltes des Wassers bis auf einen Wert von etwa 0,3 mg $O_2$/l erreichen. Bei der Vakuumentgasung wird der pH-Wert des Wassers und unter Umständen auch dessen Temperatur etwas erhöht, was sich beides ebenfalls günstig auf die Vermehrung der Biomasse und damit auf die Wirtschaftlickeit des Verfahrens auswirkt.

In weiterer Ausgestaltung der Erfindung werden als Denitrifikationsbakterien die Stämme Pseudomonas fluorescens DSM 3477 oder DSM 3478 eingesetzt und es wird dem Wasser zusätlich Phosphat $PO^{3-}$ zugesetzt. Der Zusatz von Phosphat ist notwendig, weil es sich dabei um einen unentbehrlichen Bestandteil der Bakterienzellen handelt. Bei den genannten Stämmen handelt es sich um Denitfifikationsbakterien guter Wirksamkeit, die unter leicht einzuhaltenden Bedingungen auf dem Trägermaterial einer Denitrifikationsstufe besonders gut gedeihen und die darüber hinaus den besonderen Vorteil haben, daß sie nicht pathogen sind.

Als besonders vorteilhaft für die Durchführung des erfindungsgemäßen Verfahrens hat es sich erwiesen, wenn dem Wasser als Nährstoff Ethylalkohol zugesetzt wird. Mit Ethanol wird insbesondere bei Verwendung der vorstehend genannten Bakterienstämme eine besonders gute Denitrifizierung erhalten, die nach der folgenden Gleichung abläuft:

$$7\ NO_3^- + 3{,}25\ C_2H_5OH \rightarrow 3{,}5\ N_2 + 6{,}5\ CO_2 + 8{,}25\ H_2O + OH^-$$

Gleichzeitig hat die Verwendung von Ethylalkohol als Nährstoff den Vorteil, daß er nicht toxisch ist und eine zuverlässigere Nachreinigung ermöglicht.

Bei der Durchfürung des erfindungsgemäßen Verfahrens wird als Trägermaterial für die Denitrifikationsbakterien vorteilhaft Sand mit einer Körnung im Bereich von 0,3 bis 1 mm verwendet und das Trägermaterial in der Denitrifikationsstufe von em Wasser von unten nach oben mit einer Geschwindigkeit durchströmt, bei der das Trägermaterial ein Schwebe- oder Wirbelbett bildet. Bei dem Sand kann es sich vorzugsweise um $SiO_2$ oder um Granat handeln. Je nach der Korngröße und dem specifischen Gewicht des Sandes, aber auch nach dem Grad der Bewachsung mit Biomasse, kann die Strömungsgeschwindigkeit des Wassers 8 bis 30 m/h betragen.

Die Verwendung von Sand als Trägermaterial und die Erzeugung eines Schwebe- oder Wirbeltbettes hat den besonderen Vorteil, daß für das Aufwachsen der Bakterien eine sehr große Oberfläche zur Verfügung steht und im Betrieb gewährleistet ist, daß das zu behandelnde Wasser mit den auf der Oberfläche der Sandkörnchen aufgewachsenen Bakterien in innige Berührung kommt. Hierduch wir die Wirksamkeit des erfindungsgemäßen Verfahrens bedeutend gefördert. Dabei kann die Gefahr eines Zusammenbackens der Sandkörnchen nach einem Abschalten des das Wirbelbett enthaltenden Reaktors oder auch durch übermäßiges Wachstum der Biomasse dadurch vermindet werden, daß das Trägermaterial, insbesondere vor dem Anfahren des Verfahrens, ggf. aber auch während des laufenden Betriebes, durch kurzzeitiges Hindurchleiten von Luft aufgelockert wird. Darüber hinaus ist es auch möglich, das Trägermaterial während des Betriebes des Reaktors zu rühren.

Die Kontaktzeit zwischen dem zu denitrifizierenden Wasser und den Denitrifikationsbakterien ist durch die Größe der Denitrifikationszone und der für einen einwandfreien Betrieb erforderlichen Strömungsgeschwindigkeit des Wassers bestimmt. Wenn diese Kontaktziet nicht ausreicht, um einen gewünschten Grad der Denitrifikation zu erreichen, besteht nicht nur die Möglichkeit, das Wasser nacheinaner durch mehrere hintereinander geschaltete Denitrifikationsstufen hindurchzuleiten, sondern es kann das Wasser von Ausgang der Denitrifikationsstufe an deren Eingang zurückgeführt werden, so daß es die gleiche Denitrifikationsstufe mehrfach durchläuft. Durch Bemessen des Anteiles des zurückgeführten Wassers zum zugeführten Rohwasser läßt sich praktisch jeder beliebige Denitrifikationsgrad einstellen. Eine hohe Rückführrate kann insbesondere beim Anfahren des Verfahrens von Vorteil sein, wenn die Biomasse und demgemäß die bei einem Durchgang durch die Denitrifikationsstufe erreichbare Denitrifikation noch gering ist. Um die Einlaufphase des Verfahrens zu verkürzen, kann weiterhin beim Anfahren des Verfahrens die Wassertemperatur auf 15 bis 20°C erhöht werden.

Gegenstand der Erfindung ist auch eine Vorrichtung zur Durchführung des vorstehend behandelten Verfahrens. Bekannte Vorrichtungen dieser Art, wie sie in der eingangs genannten Literaturstelle von Haberer beschrieben sind, weisen einen ein Trägermaterial mit darauf angesiedelten heterotrophen Denitrifikationsbakterien enthaltenden Schwebe- oder Wirbelbett-Reaktor, Einrichtungen zum Hindurchleiten von Wasser durch den Reaktor und zum Beimengen von Nährstoffen zum Wasser auf. Nach der Erfindung ist dem Reaktor eine Vakuum-Entgasungsstufe vorgeschaltet, die einen sehr einfachen Aufbau hat und geringe Betriebskosten verursacht. Darüber hinaus bewirkt sie nicht nur eine Reduzierung des Sauerstoffgehaltes, sondern auch des $CO_2$-Gehaltes mit einer Anhebung des pH-Wertes. Endlich findet in der Vakuum-Entgasungsstufe auch eine gewisse Erwärmung des Wassers statt. Sowohl die Erhöhung des pH-Wertes als auch die Erwärmung wirken sich günstig für das Denitrifikationsverfahren aus.

Bei einer bevorzugten Ausführungsform der Erfindung enthält der Reaktor über einem Siebboden eine Füllung aus Sand mit einer Körnung im Bereich von 0,3 bis 1 mm, und unterhalb des Siebbodens ist eine Zulaufleitung und am oberen Ende eine Ablaufleitung für das behandelte Wasser angeschlossen. Ein solcher Reaktor hat einen einfachen Aufbau und erlaubt zugleich eine sehr wirksame Durchfuhrung des

erfindungsgemäßen Verfahrens.

Obwohl es möglich ist, die Nährstoffe dem zu behandelnden Wasser im Bereich der zum Reaktor führenden Leitung beizumengen, ist es besonders vorteilhaft, den sich unterhalb des Siebbodens befindenden Raum des Behälters als Mischkammer zu verwenden und von der Zulaufleitung für das Wasser getrennte Leitungen zum Zuführen von Nährstoffen vorzusehen, welche in diesen Raum münden. Dabei ist es zweickmäßig, wenn die Leitungen für das Wasser und/oder die Nährstoffe jeweils in den Siebboden durchsetzenden Düsen enden, die eine gleichmäßige Verteilung der zugeführten Flüssigkeit auf den Behälterquerschnitt gewährleisten. So kann beispielswiese der Siebboden eine Vielzahl die Düsen bildende Bohrungen aufweisen, deren Länge eine Vielfaches ihres Durchmessers beträgt und deren Durchmesser in Strömungsrichtung im Bereich ihrer Mitte vermindert ist. Es können aber auch die Düsen dazu ausgebildet sein, das zu denitrifizierende Wasser und die Nährstoffe getrennt voneinander in einem Winkel von 70° bis 90° zur Achse des Behälters einzuspritzen. Bei einer besonders bevorzugten Ausführungsform der Erfindung sind die mit Nährstoffeitungen verbundenen Düsen oberhalb der mit der Wasserleitung verbundenen Düsen angeordnet, so daß das in sehr viel größerer Menge zugeführte Wasser die Nährstoffe aufnimmt und über den gesamten Behälter vertielt. So können beispielsweise die Wasserdüsen sternförmig angeordnete Spritzöffnungen aufweisen und die Nährstoffdüsen in er Mitte zwischen den Spritzöffnungen der Wasserdüsen angeordnet sein.

Für die Zuführung des Wassers und der Nährstoffe ist es besonders zweickmäßig, wenn der unterhalb des Siebbodens liegende Raum durch eine zum Siebboden parallele Wand in zwei Kammern unterteilt ist und die Wasser- und Nährstoffleitungen sowie die entsprechenden Düsen an je eine dieser kammern angeschlossen sind.

Besonders dann, wenn der Reaktor eine beträchtliche Höhe aufweist und die Nitratbelastung des Wassers relativ hoch ist, so daß eine einwandfreie Denitrifizierung eine relativ geringe Strömungsgeschwindigkeit des Wassers erfordert, kann des vorkommen, daß die sich im Reaktor bildende Biomasse so stark ist, daß eine Zusammenballung der Teilchen des Trägermaterials und damit eine Verstopfen der Wege für den Wasserdurchtritt stattfindet. In solchen Fällen kann eine Auflockerung des Wirbelbettes durch das Einblasen von Luft erfolgen. Daher sieht eine bevorzugte Ausführungsform der Erfindung vor, daß an das untere Ende des Behälters eine Luftleitung angeschlossen ist. Durch kurzzeitiges Zuführen von Luft kann auch beim Anfahren eines Reaktors das Aufwirbeln des Trägermaterials unterstützt werden. Ebenso ist es zweckmäßig, wenn in dem Behälter ein Rührwerk angeordnet ist, um das Austragen abgelöster Biomasse, aber auch der im Verlauf des Verfahrens freigesetzten Gase, insbesondere Stickstoff ($N_2$) und Kohlensäure ($CO_2$), aus dem Reaktor zu fördern.

Die Erfindung wird im folgenden anhand der in der Zeichnung dargestellten Ausführungsbeispiele näher beschrieben und erlaütert. Es zeigen

Fig. 1a, 1b eine schematische Darstellung einer Denifrifizierungsvorrichtung

Fig. 2 eine Diagramm zur Erläuterung der Arbeitsweise eines Schwebe/Wirbelbett-Reaktors,

Fig. 3 eine Draufsicht auf einen Abschnitt des Siebbodens des Reaktors der Vorrichtung nach Fig. 1a,

Fig. 4 einen Teilschnitt längs der Linie IV—IV durch den Siebboden nach Fig. 3,

Fig. 5 einen Querschnitt ähnlich Fig. 4 durch eine weitere Ausführungsform eines Siebbodens.

Fig. 6a, 6b eine schematische Darstellung einer Ausführungsform einer Vorrichtung zur Durchführung des erfindungsgemäßen Verfahrens,

Fig. 7 einen Querschnitt durch den unteren Abschnitt des Reaktors der Vorrichtung nach Fig. 6a und

Fig. 8 eine weitere Ausführungsform des unteren Abschnittes eines Reaktors ähnlich Fig. 7.

Hauptbestandteil der in den Fig. 1a, 1b dargestellten Vorrichtung zur Durchführungs des erfindungsgemäßen Verfahrens ist ein Reaktor 1 in Form eines zylindrischen, stehenden Behälters, der über einem Siebboden 2 eine nicht näher dargestellte Sandfüllung mit einer Körnung von 0,3 bis 0,5 mm enthält. Die Sandkörner sind mit Denitrifikations-bakterien der Stämme "Pseudomonas fluorescens" DSM 3477 und DSM 3478 besiedelt. In den Raum 3 unterhalb des Siebbodens 2 mündet eine Wasserleitung 4, die von einer Hydrogenierungsstufe 5 kommt, der das Rohwasser über eine Leitung 7 zugeführt wird. Diese Hydrogenierungsstufe 5 ist bei einer erfindungsgemäßen Vorrichtung nicht enthalten, sondern durch eine Vakuumentgasungsstufe 51 ersetzt, und wird daher nicht näher bechrieben. Das die Hydrogenierungsstufe 5 verlassende und auf der Leitung 4 dem Reaktor 1 zugeführte Wasser ist im wesentlichen frei von gelstem Sauerstoff.

An die Leitung 4 sind Behälter 11, 12, 13 angeschlossen, die es gestatten, dem dem Reaktor 1 zugeführten Wasser für den Verfahrensablauf benötigte Reagenzien beizumengen, insbesondere Nährstoffe für die im Reaktor 1 angesiedelten Bakterien. So kann der Behälter 11 Ethylalkohol und der Behälter 12 Phosphat enthalten. Die dem Wasser in der Leitung 4 zugemischten Mengen werden von einer Steuereinheit 14 bestimmt.

An den Ausgang des Reaktors 1 ist über eine Leitung 21 eine Absetzbecken 22 angeschlossen, das von einer Waschanlage 23 gefolgt wird. Von der Waschanlage 23 führt eine Leitung 24 zurück zu einer dicht über dem Siebboden 2 liegenden Stelle des Reaktors 1. Weiterhin umfaßt die Vorrichtung nach den Fig. 1a, 1b eine der Waschanlage folgende Be- und Entgasungskammer 25, an die sich ein Mehrschichtfilter 26 und eine Entkeimungsstufe 30 anschließen. Die die Be- und Entgasungskammer 25 und den Mehrschichtfilter 26 verbindende Leitung 27 ist durch eine Leitung 28 mit der das Rohwasser zuführenden Leitung 7 verbunden.

Bei dem Reaktor 1 handelt es sich um einen Schwebe/Wirbelbett-Reaktor, d.h., daß das über die Leitung 4 zugeführte Wasser durch den Siebboden 2 hindurch die im Behälter 1 enthaltene Sandmenge mit einer solchen Geschwindigkeit durchströmt, daß eine Fluidisierung eintritt. Fig. 2 zeigt ein Diagramm, das di von der Wasserstromgeschwindikeit abhängigen Zustände in einem solchen Reaktor veranschaulicht. Auf der Abszisse ist die Wasserstromgeschwindikeit V und auf der Ordinate der Druck $\triangle$ P über dem von im Behälter 1 enthaltenen Sand gebildeten Bett jeweils logarithmisch aufgetragen. Bei kleinen Geschwindigkeiten des Wasserstromes bildet der im Behälter 1 vorhandene Sand ein Festebett, d.h. daß die Sandkörner eine feste Lage haben. Mit zunehmender Wasserstromgeschwindigkeit V nimmt der Druck $\triangle$P über dem Sandbett zu, wie es der Abschnitt 31 der Kurve in Fig. 2 zeigt. Bei Erreichen einer bestimmten Geschwindigkeit Vt geht das Festbett in ein fluidisiertes Bett über, d.h., daß die Sandkörnchen vom Wasserstrom mitgenommen werden, dabei aber lediglich eine Auflockerung des Festbettes stattfindet, ohne daß die Wasserstromgeschwindigkeit groß genug ist, um die Sandkörnchen mitzureißen und aus dem Reaktor auszutragen. Im Zusatand des fluidisierten Bettes bleibt der Druck $\triangle$P über dem Bett konstant, wie es der Kurvenabschnitt 32 in Fig. 2 zeigt. Bei Erreichen einer bestimmten Wasserstromgeschwindigkeit Vh werden dann die Sandkörner des Bettes nicht nur aufgewirbelt, sondern von dem Wasserstrom mitgenommen. Es findet dann eine hydraulische Austragung statt, die von einem weiteren Anstieg des Druckes $\triangle$P begleitet ist, wie es der Kurvenabschnitt 33 in Fig. 2 zeigt. Der Reaktor 1 wird in dem durch den Kurvenabschnitt 32 gekennzeichneten Betreibszustand des fluidisierten Bettes betreiben. Die Wasserstromgeschwindikeit ist dabei von der Korngröße und dem spezifischen Gewicht des im Behälter vorhandenen Sandes abhängig. Bei der dargestellten Vorrichtung kann Quarzsand mit einer Körnung von 0,3 bis 1 mm Verwendung finden, und es hat der Wasserstrom eine Geschwindigkeit von 8 bis 30 m/h. Statt Quarzsand könnte beispielsweise auch Granat-Sand Verwendung finden. Die Reaktorhöhe kann 3,5 bis 5 m betragen. Für die Korngröße des Sandes ist maßgebend, daß Körner eine geringere Oberfläche besitzen und daher eine geringere Ansiedlungsfläche für die Denitrifikationsbakterien bieten. Die Wirksamkeit des besiedelten Wirbelbettes nimmt daher mit wachsender Korngröße ab. Zugleich sind höhere Wassergeschwindigkeiten zulässig und auch notwendig, um den Zustand der Fluidisierung zu erreichen und nicht zu überschreiten. Es wird daher je nach der Nitratbelastung des Wassers und dem gewünschten Durchsatz bei vorgegebener Baugröße des Reaktor-Behälters eine optimale Korngröße geben. Bevorzugte Korngrößen sind 0,3 bis 0,5 mm, 0,4 bis 0,6 mm und 0,5 bis 0,8 mm.

Der in Fig. 1a dargestellte Reaktor 1 ist an seinem oberen Ende mit einem Abscheider 15 für Biomasse versehen, der verhindert, daß große Mengen von aus dem Wirbelbett ausgetragener Biomasse über die Leitung 21 zum Absetzbecken 14 gelangen. Eine Rückführleitung 16 verbindet den Abscheider 15 mit einer dicht über dem Siebboden 2 liegenden Stelle des Behälters. Weiterhin ist in dem Reaktor 1 ein Rührer 17 angeordnet, und es mündet in den sich unter dem Siebboden 2 befindenden Raum 3 eine Luftleitung 18.

Für einen einwandfreien Betrieb des Reaktors 1 ist es wichtig, daß das über die Leitung 4 zugeführte und mit Nährstoffen für die Bakterien versetzte Wasser gleichmäßig auf den gesamten Behälterquerschnitt verteilt wird, damit eine gleichmäßige Durchströmung und Aufwirbelung des Sandbettes stattfindet und der gesamte Wasserstrom gleichmäßig mit den Denitrifikationsbakterien in Kontakt kommt. Es muß vermieden werden, daß im Betrieb Durchbrüche in Form von Kanälen stattfinden, durch welche hindurch das Wasser das Sandbett in einem konzentrierten Strom schnell durchströmt, ohne daß die gewünschten Reaktionen stattfinden können. Daher hat der Siebboden 2 des in Fig. 1a dargestellten Reaktors 1 die in den Fig. 3 und 4 veranschaulichte Ausbildung als Lochplatte, deren Dicke so groß ist, daß die die Lochplatte durchdringenden Bohrungen 41 eine Länge haben, die ein Vielfaches ihres Durchmessers beträgt. Zudem wiesen die Bohrungen 41 im Bereich ihrer Mitte jeweils eine Verjüngung 42 auf, derart, daß der Durchmesser der Bohrungen 41 an der oben liegenden Austrittsseite geringer ist als an der unten liegenden Eintrittsseite. Auf diese Weise wird eine Art Düsen gebildet, aus denen das Wasser in einer Vielzahl von auf den gesamten Querschnitt des Behälters 1 verteilten Strahlen austritt, die gewährleisten, daß bei ausreichend hoher Strömungsgeschwindigkeit des Wassers eine einwandfreie Aufwirbelung des Sandbettes erfolgt. Bei dem Ausführungsbeispiel nach den Fig. 3 und 4 hat die den Siebboden 2 bildende Platte eine Dicke von 30 mm, während die Bohrungen 41 einen Durchmesser von 6 bzw. 4 mm haben. Ihr Abstand beträgt im Schnitt der Fig. 4 25 mm.

Beim Betrieb der in den Fig. 1a, 1b dargestellten Vorrichtung wird das praktisch sauerstoffrei gemachte Wasser wird über die Leitung 4 der sich unter dem Siebboden 2 befindenden Kammer 3 des Reaktors zugeführt. Dabei wird das Wasser aus den Behältern 11 und 12 mit Nährstoffen für die im Reaktor enthaltenen Denitrifikationsbakterien versetzt, nämlich mit Ethylalkohol und mit Phosphat in der von der Steuereinheit 14 betimmten Menge. Das Wasser strömt dann in dem Reaktor 1 durch den Siebboden 2 nach oben und gerät mit den auf den im Wirbelbett gehaltenen Sandkörnchen angesiedelten Denitrifikationsbakterien in Kontakt. Das das Wasser keinen Sauerstoff mehr enthält, entnehmen die Bakterien den zum Atmen benötigten Sauerstoff dem im Wasser enthaltenen Nitrat und bauen dabei das Nitrat bis zu gasförmigem Stickstoff ab, der nach oben aus dem Reaktor 1 entweichen kann. Dabei werden der zugeführte Ethylalkohol und das Phosphat verbraucht. Es tritt daher aus dem Reaktor 1 über die Leitung 21 im wesentlichen nitratfreiss Wasser auf, das auch keinen störenden Gehalt an Ethylalkohol und an Phosphat mehr besitzt. In einer Versuchsanlage wurde Rohwasser mit einem Gehalt zwischen 55 und 70 mg $NO_3^-$/l Wasser mit einer Geschwindigkeit von etwa 15 bis 20 m/h durch den Reaktor geleitet. Dabei wurde der Nitratgehalt bis auf Werte von weniger als 1 mg/l vermindert.

Die Durchsatzgeschwindigkeit hängt von der Größe der Sandkörner und dem Ausmaß des Bewuchses der Sandkörnchen mit Biomasse ab. Bei stärkerem Bewuchs der Sandkörnchen genügt eine geringere Strömungsgeschwindikeit des Wassers zur Fluidisierung. Ein Rührer 17 dient dazu, die Sandkörnchen in zusätzliche Bewegung zu versetzen und bewirkt ein Ablösen von Biomasse von den Sandkörnern, Spuren von Biomasse mit Sandanteilen werden über die Leitung 21 aus dem Reaktor ausgetragen und dem Absetzbecken 22 und anschließend der Waschanlage 23 zugeführt. Hier findet eine Trennung des Wassers von dem Sand und der Biomasse statt. Der gewaschene Sand kann über die Leitung 24 in den Reaktor 1 zurückgeführt werden, während die abgetrennte Biomasse als Schlamm über die Leitung 29 aus der Waschanlage 23 abgeführt wird.

Die anschließende Be- und Entgasungskammer 25 und der Mehrschichtfilter 26 bilden Stufen, wie si in der Trinkwasseraufbereitung üblich sind und den Zweck haben, die geforderte Trinkwasserqualität zu gewährleisten.

Die mit der beschriebenen Anlage erreichbare Denitrifizierung auf einen Nitratgehalt bis weniger als 1 mg/l ist sehr viel weitergehend als es die Richtlinien fordern. Nach der EG-Norm darf der Grenzwert für Trinkwasser bis zu 50 mg/l Nitrat betragen. Der Guidewert sieht einen Nitatgehalt von ca. 25 mg/l vor. Daher ist es möglich, das nach der Erfindung fast vollständig denitrifizierte Wasser mit Rohwasser zu verschneiden, um auf einen Wert zwischen beispielsweise 20 und 25 mg Nitrat/1 Wasser zu kommen.

Wird die in den Fig. 1a und 1b dargestellte Vorrichtung in Betrieb genommen, so wird der Behälter des Reaktors 1 mit der erforderlichen Sandmenge gefüllt, die dann noch nicht mit Bakterien besiedelt ist. Anstatt auf eine Besiedelung mit den in Wasser natürlicherweise vorkommenden Denitrifikanten zu vertrauen, kann in das Sandbett eine Lösung mit Denitrifikationsbakterien der Art "Pseudomonas fluorescens" DSM 3477 und/oder DSM 3478 gegeben werden. Unter der Wirkung der mit dem dann eingeleiteten Wasser zugeführten Nährstoffe vermehren sich die bereits in großer Menge vorliegenden Bakterien der genannten Stämme sehr rasch, so daß die Vorrichtung sehr schnell zu voller Wirkung kommt. Dabei kann es nützlich sein, das Wasser zunächst über die Leitung 16 im Kreislauf zu führen und ggf. sogar über den Behälter 13 mit Natriumnitrat anzureichern, jedoch wird ein sehr schnelles Anfahren der Vorrichtung auch dann erzeilt, wenn das Wasser zunächst nur mit der zur Bildung eines Schwebe/Wirbelbettes erforderlichen Mindestgeschwindigkeit in den Reaktor eingeleitet wird. Die Ausbildung eines Schwebe/Wirbelbettes kann gerade bei geringen Strömungsgeschwindigkeiten durch Einleiten von Preßluft über die Leitung 18 begünstigt werden, da die Preßluft den das Wirbelbett bildenden Sand auflockert. Da infolge der Entziehung von Sauerstoff die Bakterien ausschließlich vom Sauerstoff des im Wasser enthaltenen Nitrates leben, findet von Anbeginn an eine wirksame Denitrifizierung statt, und es stellen sich die Bakterien ebenfalls von Anfang an auf das Aufspalten des Nitrates zur Gewinnung des für sie lebensnotwendigen Sauerstoffes ein. Unter Beobachtung des Restnitratgehaltes in dem den Reaktor verlassenden Wasser kann dabei die Strömungsgeschwindigkeit des Wassers langsam erhöht werden, bis der vorgesehene Betreibswert erreicht ist. Unter den hier beschriebenen Verhältnissen dauert das Anfahren eines Reaktors der beschriebenen Vorrichtung nur wenige Tage. Von besonderem Vorteil ist, daß der Reaktor ohne Unterbrechung in Betrieb gehalten werden kann, weil bei einem Austragen von Biomasse it Sandkörnern für ein Rückführen des aktivierten, gereinigten Sandes in den Reaktor Sorge getragen ist. Daraus ergibt sich, daß das in der erfindungsgemäßen Vorrichtung betriebene Verfahren nach der Erfindung eine zuverlässige Möglichkeit zur kostengünstigen Denitrifizierung von Trinkwasser bietet.

Fig. 5 zeigt eine Variante eines Siebbodens 43, der anstelle des Siebbodens 2 in dem den Reaktor 1 bildenden Behälter der Vorrichtung nach den Fig. 1a, 1b Anwendung finden könnte. Der Siebboden 43 besteht wieder aus einer Platte, die jedoch nicht eine Vielzahl unmittelbar als Düsen wirkender Bohrungen aufweist, sondern die mit einer geringeren Anzahl größerer Bohrungen versehen ist, in welche Verteilerdüsen 44 eingesetzt sind. Diese Verteilerdüsen 44 besitzen rohrförmige Abschnitte 45, welche die den Siebboden 43 bildende Platte durchsetzen und deren offene Enden in den Raum 3 des Reaktors 1 münden. An der mit der Sandschicht bedeckten Oberseite der Siebplatte 43 weisen die Verteilerdüsen 44 Köpfe auf, die von radialen Bohrungen 46, 47 durchsetzt sind, die bis zu dem Inneren dor Rohre 45 reichen und dadurch eine Verbindung zu dem Raum 3 des Reaktors herstellen dem das zu denitrifizierende und mit Nährstoffen versetzte Wasser zugeführt wird. Die Bohrungen 46, 47 sind sternförmig aud den Umfang der Düsenköpfe verteilt und gewährleisten eine feine Verteilung des zugeführten Wassers auf den gesamten Querschnitt des Reaktors.

Die erfindungsgemäße Vorrichtung nach den Fig. 6a und 6b weist wiederum eine Stufe 51 zum Eliminieren von Sauerstoff, einen Reaktor 52, der ein mit Denitrifizierungsbakterien besiedeltes Sandbett enthält, ein dem Reaktor 52 nachgeschaltetes Absetzbecken oder Feststoffabscheider 53, eine Waschanlage 54, eine Be- und Entgasungskammer 55, einen Mehrschichtfilter 56 und eine Entkeimungsstufe 57 auf. Insoweit stimmt diese Vorrichtung mit der Vorrichtung nach den Fig. 1a und 1b überein. Im Unterschied zu der vorstehend beschriebenen Vorrichtung ist jedoch hier die Einrichtung zum Eliminieren des Sauerstoffes als zweistufige Vakuum-Entgasungsanlage ausgebildet. Dabei wird das Wasser in der ersten Stufe 61 verdüst und in der zweiten Stufe 62 über Einbauten verrieselt. In beiden Stufen herrscht Unterdruck, wodurch eine fast vollstandige Entgasung des Wassers erzielt wird. Auch hier sinkt wieder der Sauerstoffgehalt von dem etwa 10 mg $O_2$/l betragenden Sättigungswert auf etwa 0,3 mg $O_2$l ab. Gleichzeitig wird auch die freie Kohlensäure entfernt, wodurch sich der pH-Wert des Wassers erhöht. Darüber hinaus wird auch eine Erhöhung der Temperatur des Wassers beobachtet, die für die

nachfolgende Denitrifizierung günstig ist.

Ein weiterer Unterschied zu der Vorrichtung nach den Fig. 1a, 1b besteht darin, daß der unterhab des Siebbodens 63 befindliche Raum durch eine zum Siebboden parallele Wand 64 in zwei Kammern 65, 66 unterteilt ist. Das die Vakuum-Entgasungsstufe 51 verlassende Wasser wird über die Leitung 67 unmittelbar der oberen Kammer 65 des Reaktors 52 zugeführt, von der es über in den Siebboden 63 eingesetzte Düsen 68 in den mit Sand gefüllten Raum des Reaktors gelangt. Die Düsen 68 weisen Kanäle 69 auf, deren Enden parallel zum Siebboden 63 im Reaktor 52 münden und dadurch eine gleichmäßige, sternförmige Verteilung des Wassers bewirken.

Von dem die Entgasungsstufe 51 verlassenden Wasser wird ein Teilstrom über eine Leitung 70 abgezwiegt und der unteren Kammer 66 es Reaktors 52 zugeführt. Dieser vergleichweise geringen Wassermenge werden aus Behältern 71, 72, 73 von einer Steuereinrichtung 74 bestimmte Mengen an Ethylalkohol und Phosphat als Nahrungsmittel für die Bakterien, sowie ggf. während des Anfahrens der Vorrichtung auch eine gewisse Menge Natriumnitrat, beigemischt. Aus der unteren Kammer 66 gelangt die über die Leitung 70 zugeführte Nährstofflösung dann über weitere Düsen 75 ebenfalls in den Arbeitsraum des Reaktors 52. Die weiteren Düsen 75 werden im wesentlichen von Rohren gebildet, welche mit ihren offenden Enden bis in die Kammer 66 reichen und deren mit Sprühöffnungen 76 versehene Enden die Düsen 68 durchsetzen. Demgemäß befinden sich die mit den Nährstoffleitungen verbundenen Düsen 75 oberhalb der mit der Wasserleitung verbundenen Düsen 68, welche sternförmig angeordnete Spritzdüsen aufweisen, in deren Mitte die Nährstoffdüsen 75 angeordnet sind.

Eine bemerkenswerte Besonderheit der Vorrichtung nach den Fig. 6a und 6b besteht noch darin, daß zwischen dem Reaktor 52 und dem Absetzbecken 53 ein Entkarbonisierungsreaktor 81 angeordnet ist. Wie bereits oben erwähnt, wurde das Wasser entgast und dadurch nicht nur von gelöstem Sauerstoff, sondern auch von $CO_2$ befreit. Das Ausgasen der Kohlensäure hat eine Erhöhung des pH-Wertes zur Folge, was nicht nur für die Denitifizierung günstig ist, sondern auch für eine Entkarbonisierung. Diese Entkarbonisierung erfolgt durch die Zufuhr eines basischen Stoffes über einen am unteren Ende des Reaktors 81 vorgesehenen Einlaß 82. Dabei kann es sich vornehmlich um Kalkmilch, Soda oder Natronlauge handeln. Zwar ist bei harten und sehr harten Wässern die Herabsetzung der Karbonathärte des Wassers von erheblicher Bedeutung, jedoch ist die dabei verursachte Belastung des Wassers mit Kalkmilch oder Natrium ebenfalls ungünstig, so daß bei Zusatz von Kalkmilch in der Regel auch hierfür wiederum eine Nachbehandlung erforderlich ist, die umso kostspieliger ist, je höher die Belastung des Wassers mit diesem Stoff ist. Außerdem verursachen diese Stoffe selbst erhebliche Kosten. Durch die bei dem erfindungsgemäßen Verfahren erzielte Anhebung des pH-Wertes des Wassers können die einzusetzende Menge an basischen Stoffen und die mit dem Einsatz dieser Stoffe verbundenen Schwierigkeiten bedeutend reduziert werden. Daher ist die Anordnung des Entkarbonisierungsreaktors 81 hinter dem Denitrifizierungsreaktor 52, wo der hohe pH-Wert des Wassers noch erhalten ist, besonders vorteilhaft.

Endlich ist bei der Vorrichtung nach den Fig. 6a, 6b der Be- uind Entgasungskammer auch noch eine Ozonisierungsstufe 83 zugeordnet, von der aus in die die Waschanlage 54 und die Be- und Entgasungskammer 55 verbindende Leitung 84 Ozon und in die Be- und Entgasungskammer 55 Luft eingeführt wird. Die zu Beginn des Verfahrens vorgenommene, praktisch vollständige Entgasung des Wassers macht eine besonders sorgfältige Begasung und Anreicherung des Wassers mit Sauerstoff notwendig, um ihm Trinkwasserqualität zu verliehen.

Fig. 8 zeigt eine Variante der Ausbildung des unteren Abschnittes des Denitrifikations-Reaktors 52. Auch hier ist zwar wieder der sich unterhalb des Siebbodens 91 befindende Raum durch eine dazu parallele Wand 92 in zwei Kammern 93, 94 unterteilt, jedoch ist hier die untere Kammer 94 die Wasserkammer, von der aus die Wand 92 und den Siebboden 91 durchdringende Düsen 95 in den Arbeitsraum 96 des Reaktors reichen, während von der oberen Kammer 93 ausgehende Nährstoffdüsen 97 den Siebboden 91 durchdringen. Die Wasserdüsen 95 und Nährstoffdüsen 97 sind in diesem Fall in einem vorgegebenen Raster abwechselnd angeordnet. Die Nährstoffdüsen 97 ragen weiter über den Siebboden 91 hinaus als die Wasserdüsen 95. Die Ausgangsöffnungen beider Düsen sind horizontal, so daß also das zu denitrifizierende Wasser in einer tieferen Ebene über den Querschnitt des Arbeitsraumes 96 verteilt wird als die Nährstoffmenge von den Düsen 97. Das aufsteigende Wasser nimmt daher die in her höheren Ebene verteilten Nahrungsstoffe mit, so daß eine sehr gute Vermischung der Nahrungsstoffe in dem Wasser stattfindet.

Wie bereits erwähnt, können als Denitrifikationsbakterien bei dem erfindungsgemäßen Verfahren Bakterien der Stämme "Pseudomonas fluorescens" DSM 3477 und/oder DSM 3478 Verwendung finden. Diese Bakterienstämme zeichnen sich durch eine besonders hohe Effektivität bei der Denitrifikation, gute Beständigkeit und gutes Wachstum aus. Dazu ist von besonderer Bedeutung, daß diese Bakterienstämme keine pathogenen Eigenschaften haben. Ihre Verwendung im Trinkwasser ist daher unproblematisch.

Die genannten Bakterien wurden gewonnen, indem Grundwasser unter Zusatz von Nahrungsstoffen im Kreislauf und bei steigender Temperatur durch den Reaktor geleitet wurde. Dabei siedelten sich im Wasser enthaltene Bakterien an, und es trat langsam eine Denitrifizierung ein. Das Verfahren wurde dann unter Zusatz immer größerer Anteile von frischem, mit Nahrungsstoffen versetztem Rohwasser fortgesetzt, bis eine dichte Besiedelung des Sandbettes mit Biomasse und eine im wesentlichen konstante Denitrifizierung zu beobachten war. Die Untersuchung der Biomasse ließ unterschiedliche Stämme

erkennen, die alle der Art "pseudomonas fluorescens" zugeordnet werden konnten. Es wurden aus der in der beschriebenen Weise erhaltenen Bakterienflora zwei Stämme isoliert und be der deutschen Sammlung von Mikroorganismen hinterlegt; sie erhielten die Nummer DSM 3477 und DSM 3478.

Bei Anwendung des erfindungsgemäßen Verfahrens braucht es nicht dem Zufall überlassen werden, welche Bakterien sich auf den Trägerstoffen des Denitrifizierungs-Reaktors ansiedeln, sondern es können die Trägerstoffe mit den Denitrifikationsbakterien der Stämme "Pseudomonas fluorescens" DSM 3477 und/oder DSM 3478 geimpft werden, und zwar vorzugsweise mit einer Mischung dieser Stämme, so daß von vornherein eine große Menge wirksamer Denitrifikationsbakterien vorliegt, die sich unter den bei der Trinkwasser-Denitrifikation herrschenden Bedingungen optimal entwickeln und daher der Entwicklung anderer Bakterien keinen nennenswerten Raum mehr lassen. Dadurch werden mit dem erfindungsgemäßen verfahren unter optimalen Bedingungen bestmögliche Ergebnisse erzielt.

In allen Fällen ist die Eliminierung des Sauerstoffes mittels Vakuumentgasung aus dem zu behandelnden Wasser vor der Kontaktierung mit den Denitrifikationsbakterien von grundlegender Bedeutung. Darüber hinaus ist die Verwendung von Denitrifikationsbakterien der Stämme "Pseudomonas fluorescens" DSM 3477 und/oder DSM 3478 für die Wirksamkeit des erfindungsgemäßen Verfahrens, und dabei insbesondere die Besiedelung eines frischen Schwebe/Wirbelbettes mit gezüchteten Bakterien dieser Art, für einen hohen Wirkungsgrad des Verfahrens von besonderem Vorteil.

**Patentansprüche**

1. Verfahren zur biologischen Denitrifizierung von Wasser zur Aufbereitung von Trinkwasser unter Verwendung eines Schwebe- oder Wirbelbettreaktors mit einer Denitrifikationsstufe, die ein Trägermaterial mit darauf angesiedelten heterotrophen Denitrifikationsbakterien enthält, bei welchem Verfahren das aufzubereitende Wasser mit Nährstoffen für die Denitrifikationsbakterien versetzt und dann durch die Denitrifikationsstufe hindurchgeleitet wird, dadurch gekennzeichnet, daß das Wasser vor dessen Hindurchleiten durch die Denitrifikationsstufe einer Vakuumentgasung unterworfen wird.

2. Verfahren nach Anspruch 1, dadurch gekennzeichnet, daß durch die Vakuumentgasung eine Verminderung des Sauerstoffgehaltes des Wassers bis auf einen Wert von etwa 0,3 mg $O_2$/l erfolgt.

3. Verfahren nach Anspruch 1 oder 2, dadurch gekennzeichnet, daß man als Denitrifikationsbakterien die Stämme Pseudomonas fluorescens DSM 3477 oder DSM 3478 einsetzt und dem Wasser zusätzlich Phosphat ($PO_4^-$) zusetzt.

4. Verfahren nach einem der vorhergehenden Ansprüche, dadurch gekennzeichnet, daß dem Wasser als Nährstoff Ethylalkohol zugesetzt wird.

5. Verfahren nach einem der vorhergehenden Ansprüche, dadurch gekennzeichnet, daß als Trägermaterial dür die Denitrifikationsbakterien Sand mit einer Körnung im Bereich von 0,3 bis 1 mm verwendet wird.

6. Verfahren nach Anspruch 5, dadurch gekennzeichnet, daß die Strömungsgeschwindigkeit des Wassers 8 bis 30 m/h beträgt.

7. Verfahren nach Anspruch 5 oder 6, dadurch gekennzeichnet, daß das Trägermaterial, insbesondere vor dem Anfahren des Verfahrens, durch kurzzeitiges Hindurchleiten von Luft aufgelockert wird.

8. Verfahren nach einem der Ansprüche 5 bis 7, dadurch gekennzeichnet, daß das Trägermaterial gerührt wird.

9. Vorrichtung zur Durchführung des Verfahrens nach einem der Ansprüche 1 bis 8, mit einem ein Trägermaterial mit darauf angesiedelten Denitrifikationsbakterien enthaltenden Schwebe- oder Wirbelbettreaktor und Einrichtungen zum Hindurchleiten von Wasser durch den Reaktor und zum Beimengen von Nährstoffen zum Wasser, dadurch gekennzeichnet, das dem Reaktor (1; 52) eine Vakuum-Entgasungsstufe (51) vorgeschaltet ist.

10. Vorrichtung nach Anspruch 9, dadurch gekennzeichnet, daß der Reaktor (1; 52) über einem Siebboden (2; 63) eine Füllung aus Sand mit einer Körnung im Bereich von 0,3 bis 1 mm enthält und daß unterhalb des Siebbodens (2; 63) eine Zulaufletiung (4; 67) und am oberen Ende eine Ablaufleitung (21, 85) für das behandelte Wasser angeschlossen ist.

11. Vorrichtung nach Anspruch 10, dadurch gekennzeichnet, daß in den sich unterhalb des Siebbodens (63) befindenden Raum des Behälters voneinander und von der Zufuhrleitung (67) für das Wasser getrennte leitungen (70) zum Zuführen von Nährstffen münden.

12. Vorrichtung nach Anspruch 10 oder 11, dadurch gekennzeichnet, daß die Leitungen (4; 67, 70) für das Wasser und/oer die Nährstoffe jewils in den Siebboden (2; 63) durchsetzenden Düsen enden, die eine gleichmäßige Verteilung der zugeführten Flussigkeit auf den Behälterquerschnitt gewährleisten.

13. Vorrichtung nach Anspruch 12, dadurch gekennzeichnet, daß der Siebboden (2) eine Vielzahl die Düsen bildende Bohrungen (41) aufweist, deren Länge eine Vielfaches ihres Durchmessers beträgt und deren Durchmesser in Strömungsrichtung im Bereich ihrer Mitte vermindert ist.

14. Vorrichtung nach Anspruch 12, dadurch gekennzeichnet, daß die Düsen dazu ausgebildet sind, das zu denitrifizierende Wasser und die Nährstoffe getrennt voneinander in einem Winkel von 70° bis 90° zur Achse des Behälters einzuspritzen.

15. Vorrichtung nach einem der Ansprüche 12 bis 14, dadurch gekennzeichnet, daß die mit Nährstoffleitungen (70) in Verbindung stehenden Düsen (75) oberhalb der mit der Wasserleitung (67) in

Verbindung stehenden Düsen (68) angeordnet sind.

16. Vorrichtung nach Anspruch 15, dadurch gekennzeichnet, daß die Wasserdüsen (68) sternförmig angeordnete Spritzöffnungen (69) aufweisen und die Nährstoffdüsen (75, 76) in der Mitte zwischen den Spritzöffnungen (69) der Wasserdüsen angeordnet sind.

17. Vorrichtung nach einem der Ansprüche 11 bis 16, dadurch gekennzeichnet, daß der unterhalb des Siebbodens (63) liegende Raum durch eine zum Siebboden parallele Wand (64) in zwei Kammern (65, 66) unterteilt ist und die Wasser- und nährstoffleitungen (67, 70) sowie die entsprechenden Düsen (68, 75) an je eine dieser Kammern angeschlossen sind.

18. Vorrichtung nach einem der Ansprüche 11 bis 17, dadurch gekennzeichnet, daß an das untere Ende des Reaktors (1) eine Luftleitung (18) angeschlossen ist.

19. Vorrichtung nach einem der Ansprüche 11 bis 18, dadurch gekennzeichnet, daß in dem Reaktor (1) ein Ruhrwerk (17) angeordnet ist.

**Revendications**

1. Procédé de dénitrification bioligique d'eau pour le traitement d'eau potable par utilisation d'un réacteur à lit flottant ou fluidisé, comprenant une étape de dénitification qui contient une matière de support avec des bactéries de dénitrification hétérotrophes qui la colonisent, procédé dans lequel l'eau à traiter est additionnée de substances nutritives pour les bactéries de dénitrification et est ensuite passée au travers de l'étape de dénitrification, caractérisé en ce que l'eau est, avant son passage au travers del'étape de dénitrification, soumise à un dégazage sous vide.

2. Procédé suivant la revendication 1, caractérisé en ce qu'une réduction de la teneur en oxygène de l'eau jusqu'à une valeur d'environ 0,3 mg de $O_2$/l est produite par le dégazage sous vide.

3. Procédé suivant l'une revendications 1 et 2, caractérisé en ce que, comme bactéries de dénitrification, on met en oeuvre les souches de Pseudomonas fluorescens DSM 3477 ou DSM 3478 et on ajoute à l'eau en supplément du phosphate ($PO_4^{-}$).

4. Procédé suivant l'un des revendications précédentes, caractérisé en ce qu'on ajoute à l'eau, comme substance nutritive, de l'alcool éthylique.

5. Procédé suivant l'une des revendications précédentes, caractérisé en ce que, comme matière de support pour les bactéries de dénitrification, on utilise de sable présentant une granulation de l'ordre de 0,3 à 1 mm.

6. Procédé suivant la revendication 5, caractérisé en ce que la vitesse d'écoulement de l'eau est de 8 à 30 m/heure.

7. Procédé suivant l'une des revendications 5 et 6, caractérisé en ce que la matière de support est désagrégée par un passage de courte durée d'air, en particulier avant le démarrage du procédé.

8. Procédé suivant l'une des revendications 5 à 7, caractérisé en ce que la matière de support est agitée.

9. Dispositif pour la mise en oeuvre du procédé suivant l'une des revendications 1 à 8, comprenant un réacteur à lit flottant ou fluidisé contenant une matière de support avec des bactéries de dénitrification qui la colonisent et des dispositifs pour faire passer l'eau au travers du réacteur et pour mélanger à l'eau des substances nutritives, caractérisé en ce qu'un étage de dégazage sous vide (51) est monté en amont du réacteur (1; 52).

10. Dispositif suivant la revendication 9, caractérisé en ce que le réacteur (1; 52) contient au-dessus d'un fond perforé (2; 63) un remplissage de sable présentant une granulation de l'ordre de 0,3 à 1 mm et en ce qu'un conduit d'amenée (4; 67) est raccordé en dessous du fond perforé (2; 63) et un conduit de sortie (21, 85) pour l'eau traitée est raccordé à l'extrémité supérieure.

11. Dispositif suivant la revendication 10, caractérisé en ce que les conduits (70) d'amenée des substances nutritives, qui sont séparés l'un de l'autre et du conduit d'amenée (67) de l'eau, débouchent dans la chambre du réservoir située en dessous du fond perforé (63).

12. Dispositif suivant l'une des revendications 10 et 11, caractérisé en ce que les conduits (4; 67, 70) prévus pour l'eau et/ou les substances nutritives se terminent chacun dans des tuyères qui traversent le fond perforé (2; 63) et qui garantissent une distribution uniforme du liquide amené sur la section transversale du réservoir.

13. Dispositif suivant la revendication 12, caractérisé en ce que le fond perforé (2) présente plusieurs trous (41) qui forment les tuyères et dont la longueur est égale à une multiple de son diamètre et dont le diamètre est réduit dans le sens de l'écoulement dans la zone de leur centre.

14. Dispositif suivant la revendication 12, caractérisé en ce que les tuyères sont réalisées de façon à pulvériser l'eau à dénitrifier et les substances nutritives de façon mutuellement séparée, sous un angle de 70° à 90° par rapport à l'axe du réservoir.

15. Dispositif suivant l'une des revendications 12 à 14, caractérisé en ce que les tuyères (75), qui sont en communication avec des conduits à substance nutritive (70), sont disposées au-dessus des tuyères (68), qui sont en communication avec le conduit à eau (67).

16. Dispositif suivant la revendication 15, caractérisé en ce que le tuyères à eau (68) présentent des orifices de pulvérisation (69) agencés en étoile et en ce que les tuyères à substance nutritive (75, 76) sont agencées au centre des orifices de pulvérisation (69) des tuyères à eau.

17. Dispositif suivant l'une des revendications 11 à 16, caractérisé en ce que la chambre située en

dessous du fond perforé (63) est subdivisée en deux compartments (65, 66) par une paroi (64) parallèle au fond perforé et en ce que les conduits à eau et à substance nutritive (67, 70) ainsi que les tuyères correspondantes (68, 75) sont chaque fois raccordés à un de ces compartiments.

18. Dispositif suivant l'une des revendications 11 à 17, caractérisé en ce qu'un conduit à air (18) est raccordé à l'extrémité inférieure du réacteur (1).

19. Dispositif suivant une des revendications 10 à 18, caractérisé en ce qu'un dispositif d'agitation (17) est agencé dans le réacteur (1).

**Claims**

1. Process for the biological denitrification of water for the preparation of drinking water using a floating or vortex fluid bed reactor having a denitrification stage which contains a carrier material with heterotropic denitrification bacteria settled thereon, in the case of which process the water to be prepared is mixed with nutrients for the denitrification bacteria and is then led through the denitrification stage, characterised in that the water is subjected to a vacuum degassing step before it is led through the denitrification stage.

2. Process according to Claim 1, characterised in that the oxygen content of the water is reduced to a value of approximately 0.3 mg $O_2$/l as a result of the vacuum degassing step.

3. Process according to Claim 1 or 2, characterised in that strains of Psuedomonas fluorescens DSM 3477 or DSM 3478 are used as denitrification bacteria and phosphate ($PO_4^-$) is additionally added to the water.

4. Process according to any one of the preceeding Claims, characterised in that ethyl alcohol is added to the water as a nutrient.

5. Process according to any one of the preceeding Claims, characterised in that sand having a granularity in the region of 0.3 to 1mm is used a carrier material for the denitrification bacteria.

6. Process according to Claim 5, characterised in that flow velocity of the water is 8 to 30 m/h.

7. Process according to Claim 5 or 6, characterised in that the carrier material is aerated by air being passed through for a short amount of time in particular before the process starts.

8. Process according to Claim 5 or 7, characterised in that the carrier material is agitated.

9. Device for performing the process according to any one of Claims 1 to 8, having a floating or vortex fluid bed reactor containing a carrier material with denitrification bacteria settled thereon and devices for passing water through the reactor and for adding nutrients to the water, characterised in that a vacuum degassing stage (51) is connected upstream of the reactor (1, 52).

10. Device according to Claim 9, characterised in that the reactor (1, 52) contains a filling of sand having a granularity in the vicinity of 0.3 to 1 mm above a perforated base (2, 63); and in that a delivery pipe (4, 67) is connected below the perforated base (2, 63) and a discharge pipe (21, 85) for the treated water is connected to the upper end.

11. Device according to Claim 10, characterised in that nutrient delivery pipes (70), which are separate from one another and from delivery pipe (67), run into the container space located below the perforated base (63).

12. Device according to Claim 10 or 11, characterised in that the pipes (4: 67, 70) for the water and/or the nutrients in each case terminate in nozzles penetrating the perforated base (2: 63) and ensuring a uniform distribution of the fluid supplied over the cross-section of the container.

13. Device according to Claim 12, characterised in that the perforated base (2) comprises a plurality of bores (41) which form the nozzles and of which the length is a multiple of the diameter thereof and of which the diameter is reduced in the vicinity of the centre thereof in the flow direction.

14. Device according to Claim 12, characterised in that the nozzles are constructed such that the water to be denitrificated and the nutrients are injected separately from one another at an angle of 70° to 90° with respect to the container axis.

15. Device according to any one of Claims 12 to 14, characterised in that nozzles (75) connected to nutrient pipes (70) are disposed above the nozzles (68) connected to the water pipe (67).

16. Device according to claim 15, characterised in that the water nozzles (68) comprise spraying apertures (69) which are disposed in a star shaped manner, and the nutrient nozzles (75, 76) are disposed in the centre between the spraying apertures (67) of the water nozzles.

17. Device according to any one of Claims 11 to 16, characterised in that the space lying below the perforated base (63) is divided by a wall (64), parallel to the perforated base, into two chambers (65, 66) and the water and nutrient pipes (67, 70) as well as the corresponding nozzles (68, 75) are each connected to one of these chambers.

18. Device according to any one of Claims 11 to 17, characterised in that an air pipe (18) is connected to the lower end of the reactor (1).

19. Device according to any one of Claims 10 to 18, characterised in that a stirring apparatus (17) is disposed in the reactor (1).

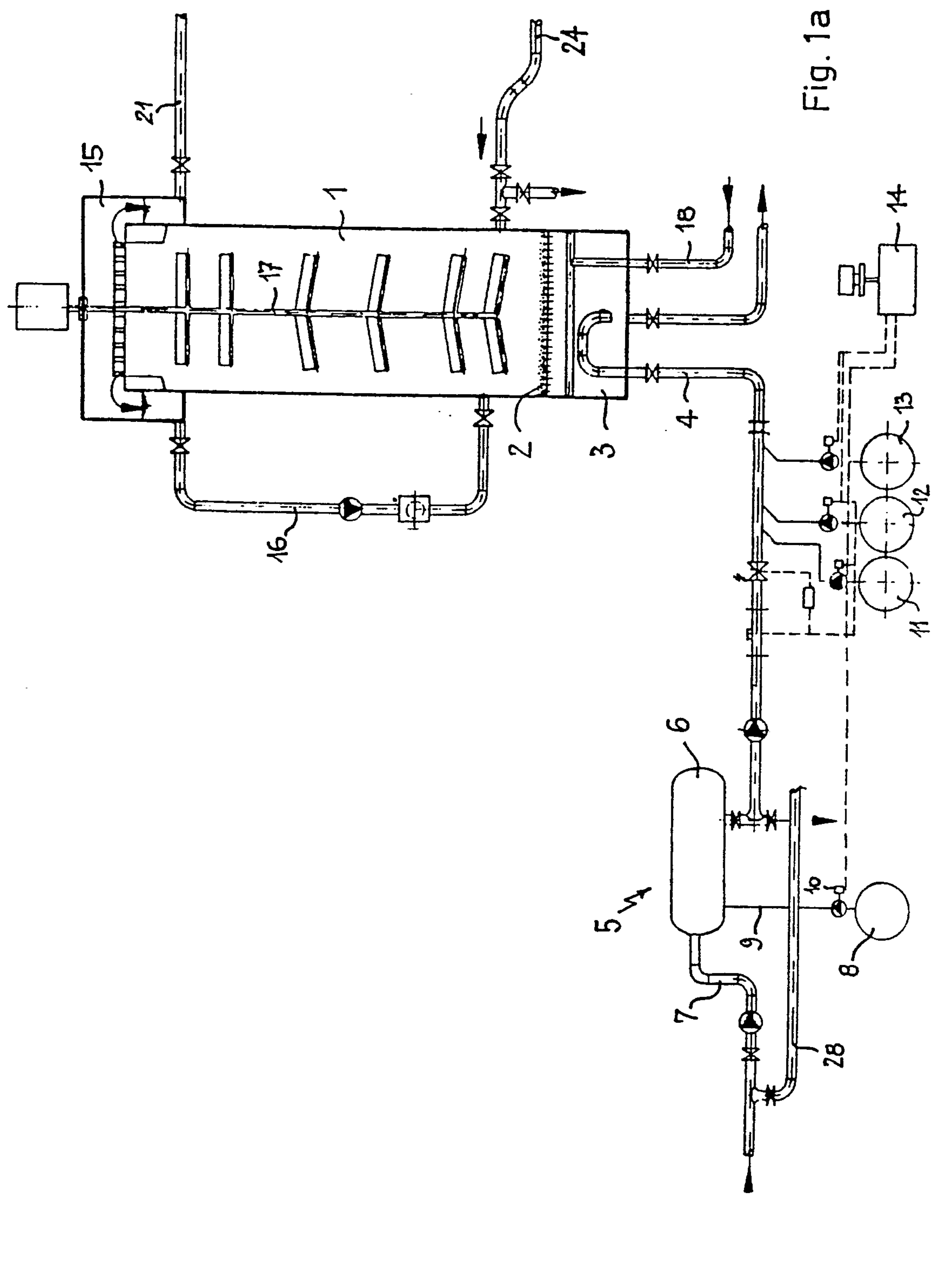


Fig. 1a

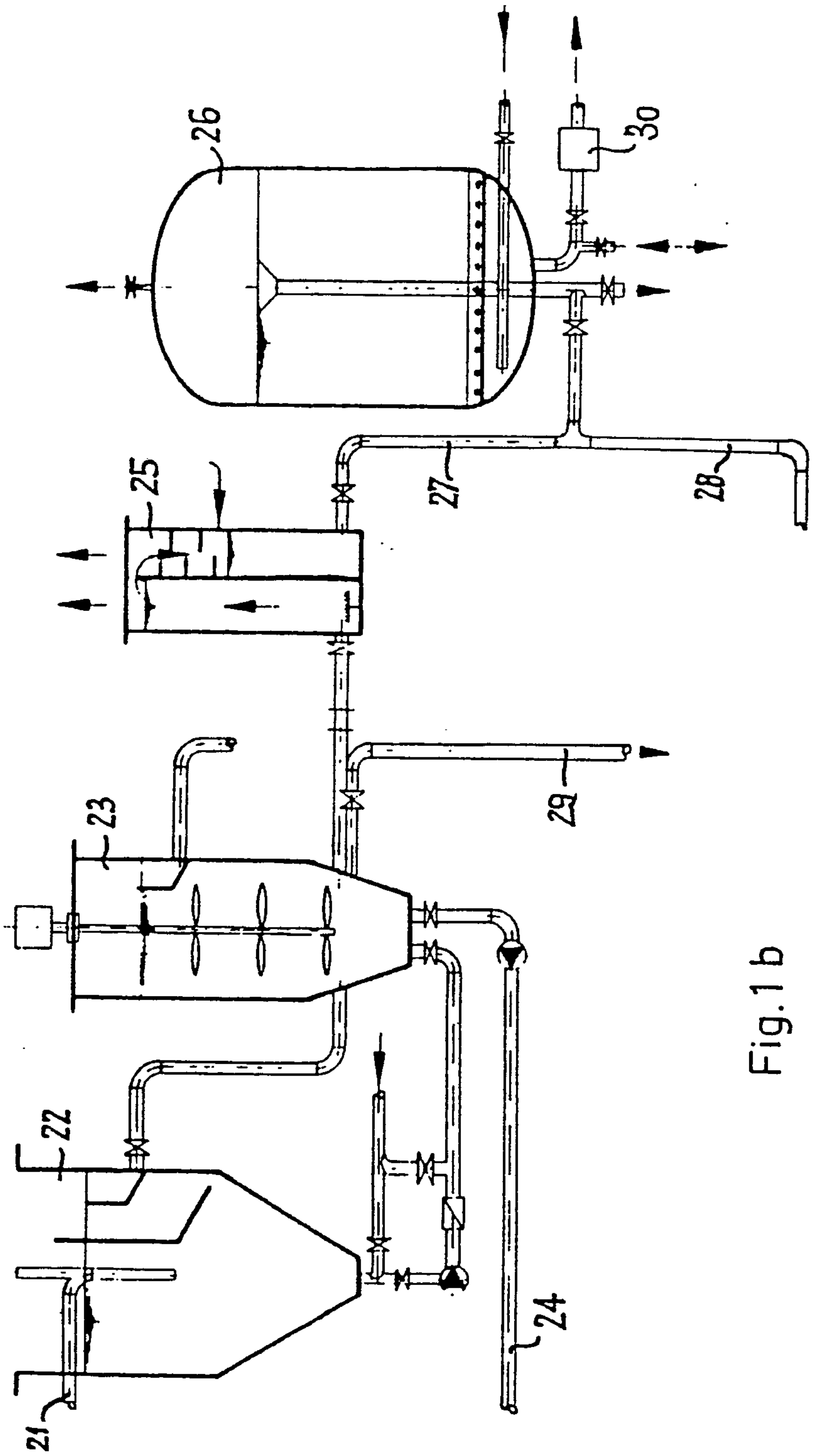


Fig.1b

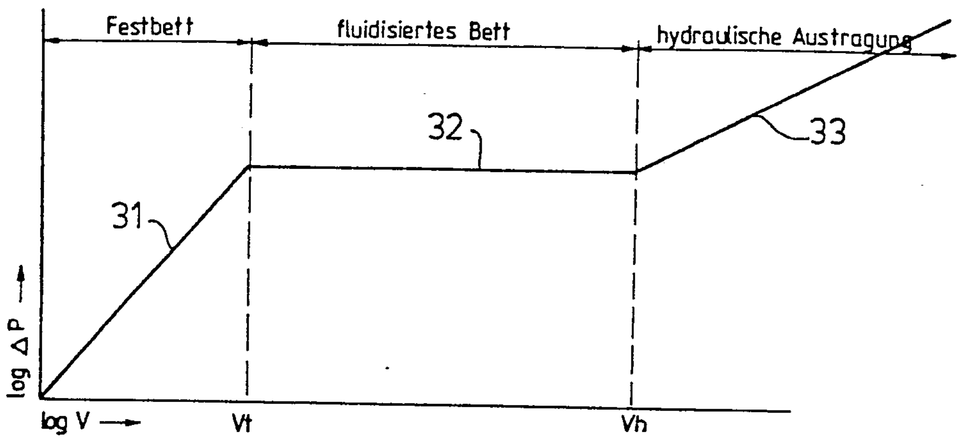


Fig. 2

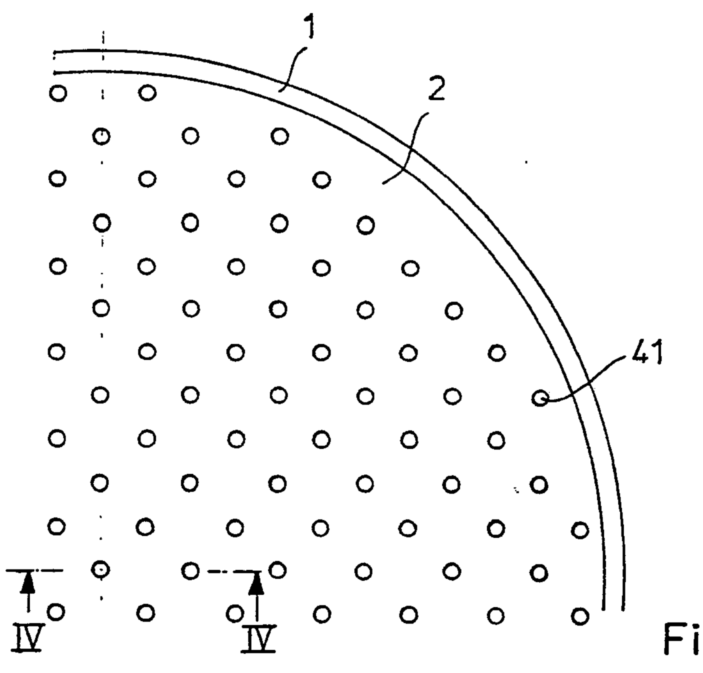


Fig. 3

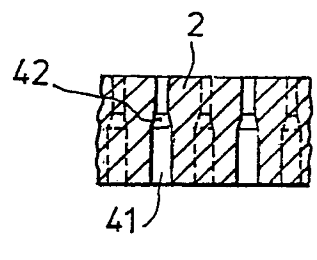


Fig. 4

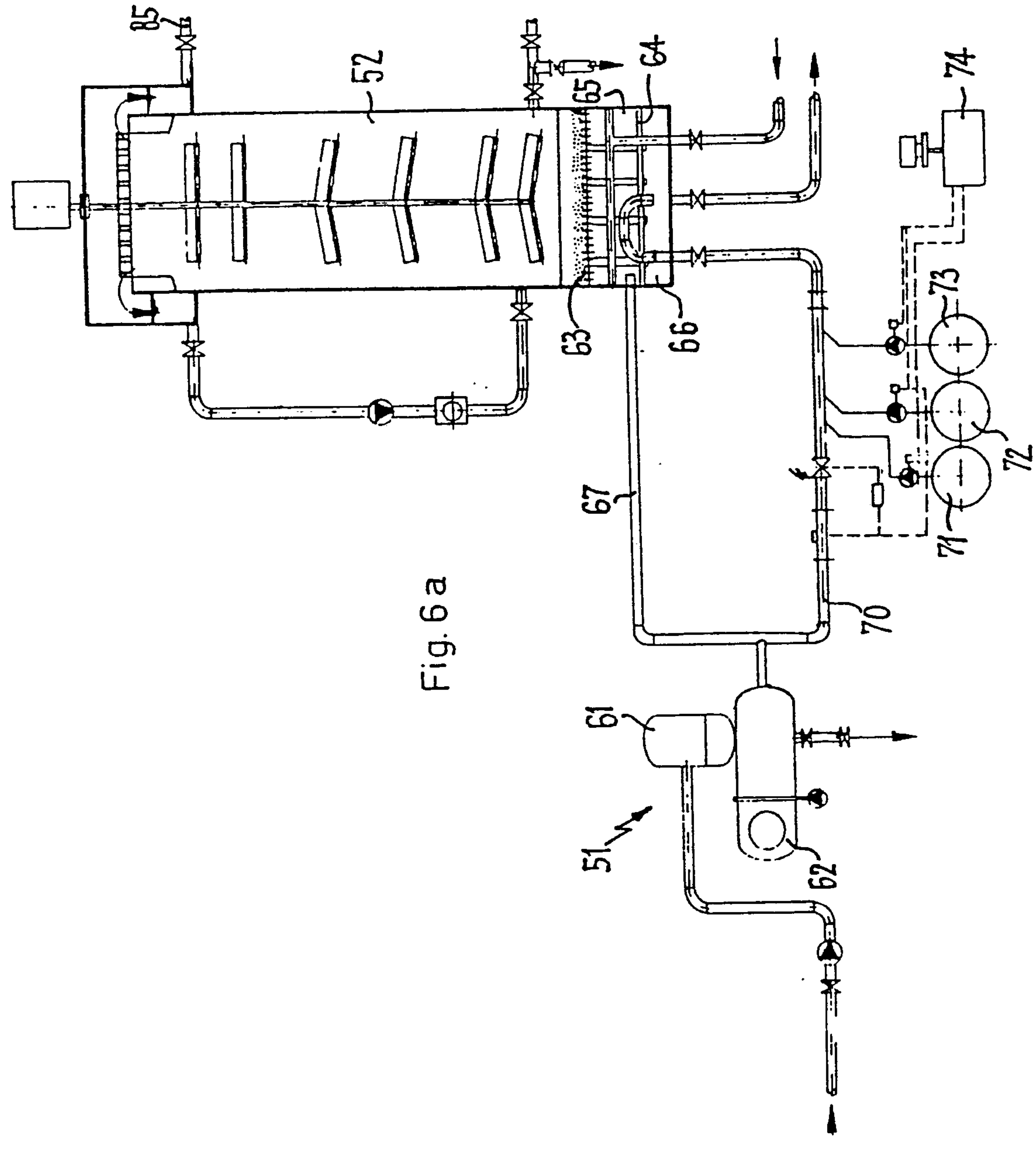


Fig. 6a

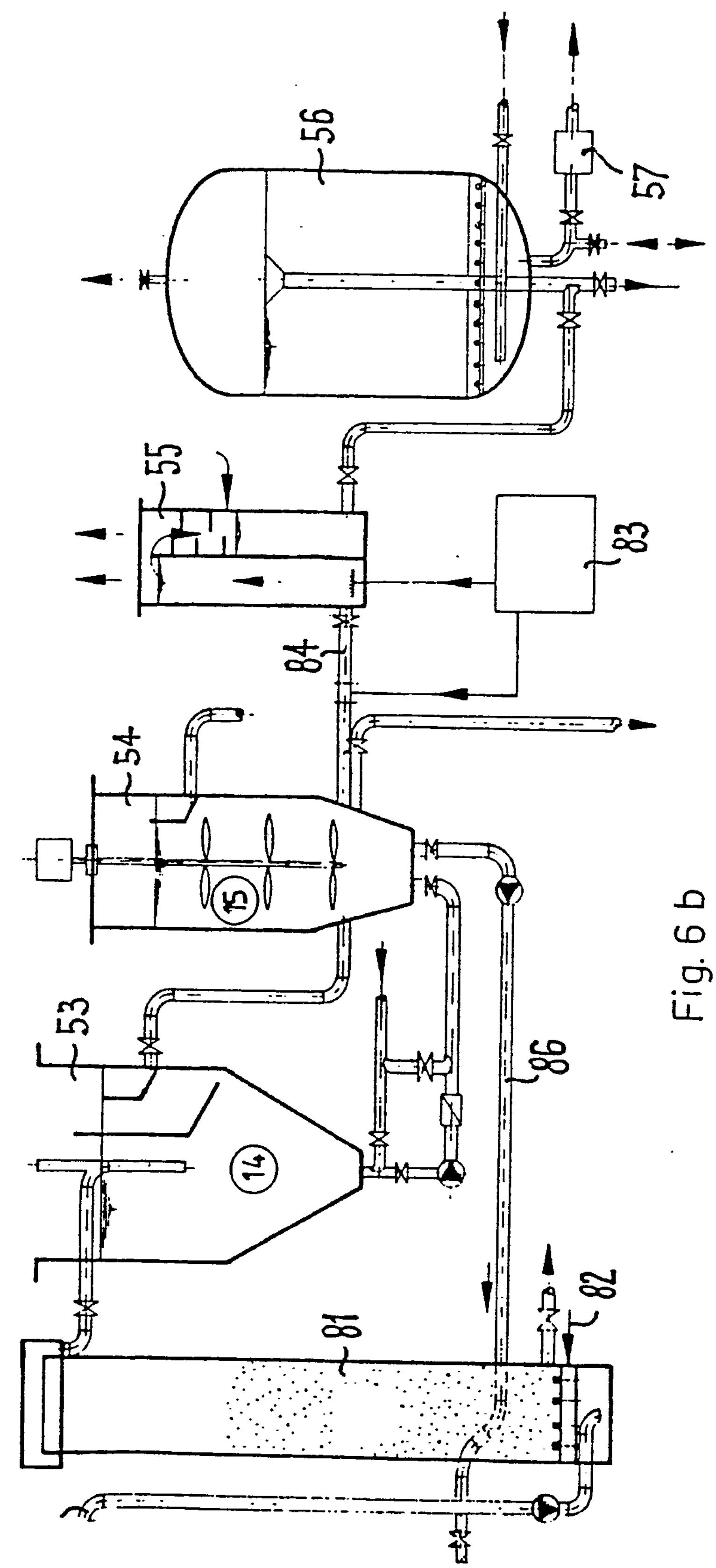


Fig. 6 b

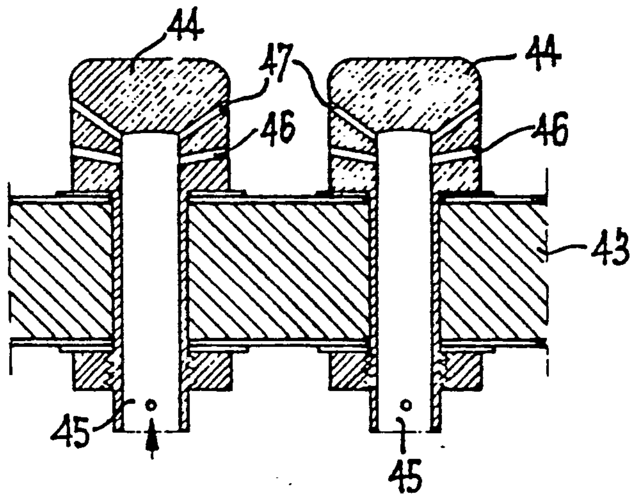


Fig. 5

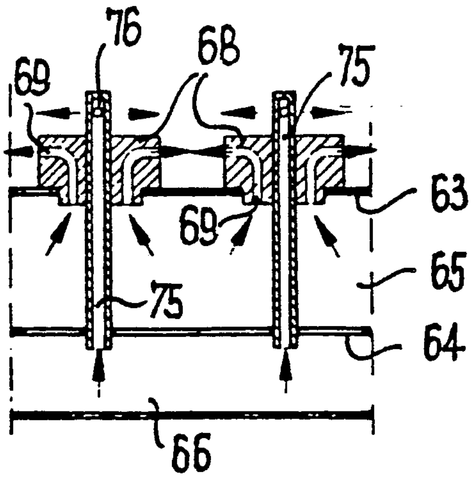


Fig. 7

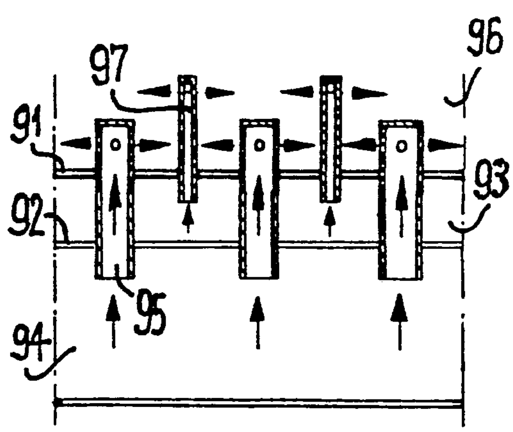


Fig. 8